# EUROPEAN PATENT SPECIFICATION

(11) **EP 2 684 545 B1**
(45) Date of publication and mention of the grant of the patent: **05.10.2016**
(21) Application number: 12755705.6
(22) Date of filing: 08.02.2012
(51) Int. Cl.: A61F 2/82

(54) **STENT**
STENT
ENDOPROTHÈSE

(30) Priority: 08.03.2011 JP 2011050555
(43) Date of publication of application: 15.01.2014
(73) Proprietor: Piolax Medical Devices, Inc., Yokohama-shi Kanagawa 240-0023 (JP)
(72) Inventor: TOYOKAWA, Yoshihide, Yokohama-shi Kanagawa 240-0023 (JP)
(74) Representative: Grünecker Patent- und Rechtsanwälte PartG mbB
(86) International application number: PCT/JP2012/052840
(87) International publication number: WO 2012/120953

(56) References cited:
- EP-A1- 2 177 181
- WO-A1-2010/077676
- WO-A2-2007/002933
- JP-A- H11 508 152
- JP-A- 2010 094 510
- JP-A- 2010 521 217
- US-A1- 2001 044 650
- US-A1- 2010 010 619

## Description

### Technical Field

The present invention relates to a stent which is detained in a tubular organ such as a blood vessel, the ureter, a bile duct, the trachea, etc. to thereby prevent the tubular organ from being narrowed or blocked and prevent an aneurysm from being ruptured or the like.

### Background Art

In recent years, there has been performed a treatment technique using a stent such that the stent is detained in a narrowed or blocked area of a human body's tubular organ such as a blood vessel, the ureter, a bile duct, the trachea, etc. to expand the narrowed or blocked area, or such that the stent is detained in a place of a generated aneurysm to prevent the aneurysm from being ruptured.

Generally, the aforementioned stent is roughly classified into a stent formed into a tubular shape by braiding metal wire and a stent formed into a tubular shape having a zigzag or frame-like linear pattern by processing the circumferential wall of a metal cylinder by laser, etching or the like.

For example, WO- 2008-001865-A discloses a covered stent including a tubular stent, and a cover member provided in an outer circumferential portion of the stent. An embodiment thereof mentions a knitted work such as a stocking as the cover member, and mentions a stent made of wire or a stent provided with a tubular flexible body having a web structure as the stent. US 2010/0010619 A1 describes a stent having the features of the preamble of claim 1. Said stent comprises a fist stent body and a second stent body, whereby the second stent body is a tubular stent at least partially disposed within the first stent body, which is a rolled stent, so that a first end of the first stent body overlaps a second end of the fist stent body for a first overlap length.

WO 2007/002933 discloses a multi-functional, thin-walled tubular structure. The device may be used as a stent or similar supporting structure, as a prosthetic intima, re-lining a vessel wall. **Summary of Invention**

### Technical Problem

Generally, a stent is contained inside a catheter, a sheath or the like in an extended and diametrically-contracted state so that the stent is conveyed to a target position. A stent formed by braiding metal wire may cause a phenomenon called "shortening" in which the axial length of the stent becomes shorter when the stent diametrically expands as a result of being released from the catheter or the like in the target position, as compared with the diametrically-contracted state. When such shortening occurs, the stent may be displaced to make it difficult to detain the stent in a desired position accurately.

On the other hand, it has been known that the aforementioned shortening hardly occurs in a stent formed by processing a metal cylinder. However, when such stent is detained in a bent tubular organ, corner portions of the zigzag or frame-like linear patterns may protrude outward. For example, the stent formed by processing a metal cylinder is configured so that zigzag or frame-like linear patterns are connected axially through connection portions. Specifically, when the stent having five or more of such connection portions in a circumferential direction is bent, the lumen of the stent may easily collapse to thereby cause the connection portions to protrude outward. In such a case, because it may easily damage the tubular organ and the corner portions may easily stick into the inner wall of the tubular organ, recovery of the stent may be difficult.

Although Patent Literature 1 mentions covering the outer circumference of the stent with a knitted work such as a stocking, since the stocking is soft and low in rigidity, it may be difficult to prevent the corner portions of the linear patterns of the stent from protruding outward.

An object of the invention is to provide a stent in which shortening at the time of detention can be prevented while outward protrusion can be prevented when the stent is detained in a bent tubular organ.

### Solution to Problem

To achieve the foregoing object, the invention provides a stent, including:
an inner stent which is formed by processing a metal cylinder so that circumferential direction units are axially connected to one another through plural connection portions and so that each of the circumferential direction units is formed of a circumferentially-extended and annularly-connected zigzag portion or of circumferentially-connected plural frame-like portions; and
an outer stent which is formed cylindrically by braiding metal wire and which is put on an outer circumference of the inner stent
wherein the inner stent and the outer stent are fixed by a resin fixing layer.

The invention may provide the stent,
wherein the outer stent has
portions where the metal wire is braided densely, and
portions where the metal wire is braided sparsely, when the outer stent is viewed along the axial direction thereof.

The invention may provide the stent,
wherein a cover member made of a resin layer is fixed to an inner circumference of the inner stent and/or an outer circumference of the outer stent.

### Advantageous Effects of Invention

According to the invention, shortening at the time of detention in a tubular organ can be prevented by the inner stent made of a metal cylinder the total length of which hardly varies between the diametrically-contracted state and the diametrically-expanded state so that the stent can be detained in a target place of the tubular organ without displacement. Because the outer stent formed by braiding metal wire which hardly protrudes outward even when the outer stent is bent is put on the outside of the inner stent, even when the stent is detained in a bent tubular organ, it can be detained while the inner stent can be prevented from protruding outward.

### Brief Description of Drawings

[Fig. 1] An exploded perspective view showing an embodiment of a stent according to the invention.
[Fig. 2] A perspective view of the stent.
[Fig. 3] (a) is a development view showing an inner stent as a constituent member of the stent, and (b) is a development view of the inner stent in another example.
[Fig. 4] A development view of an outer stent as a constituent member of the stent.
[Fig. 5] (a) is a sectional view of important part of the stent, and (b) is a sectional view of important part of another example.
[Fig. 6] (a) is an explanatory view in the case where the stent is in the diametrically-expanded state, and (b) is an explanatory view in the case where the stent is in the diametrically-contracted state.
[Fig. 7] An explanatory view in the case where the stent is detained in a bent tubular organ.

### Description of Embodiments

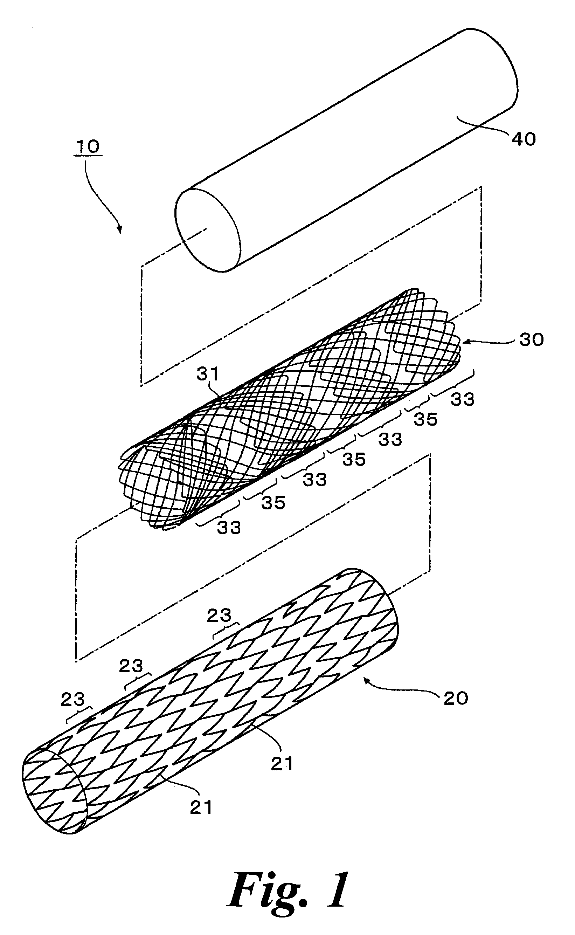

An embodiment of a stent according to the invention will be described below with reference to Figs. 1 to 7.

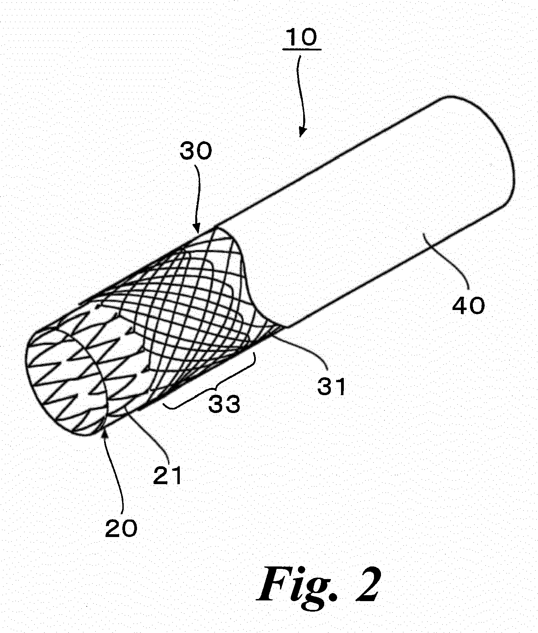

As shown in Figs. 1 and 2, the stent 10 in this embodiment has an inner stent 20, an outer stent 30 put on an outer circumference of the inner stent 20, and a cover member 40 which covers the two stents 20 and 30.

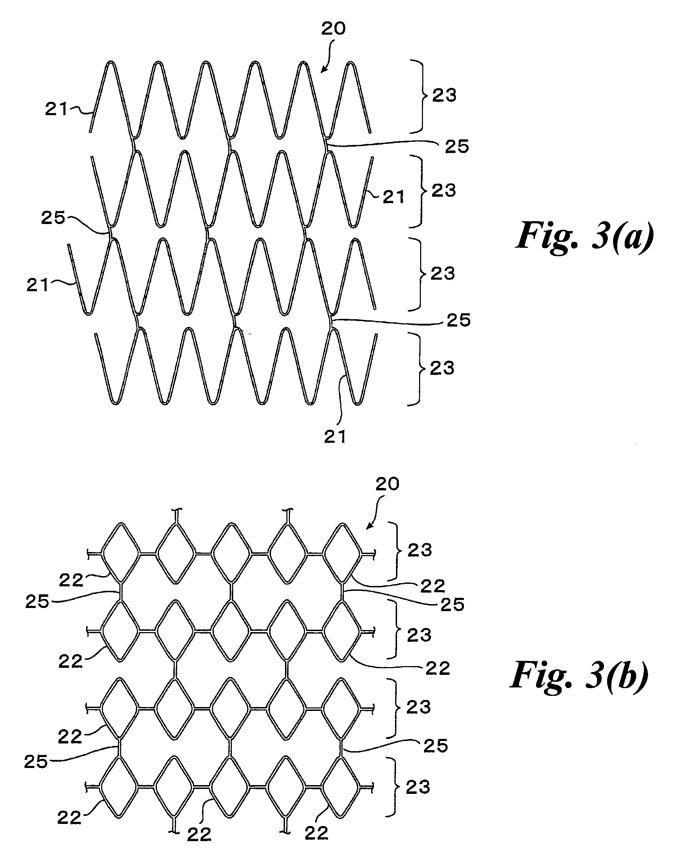

The inner stent 20 is formed by processing a metal cylinder. As shown in the development view of Fig. 3(a), a metal cylinder is processed by laser processing, etching or the like to be corrugated and extended zigzag along the circumferential direction to thereby form zigzag portions 21. Both ends of each zigzag portion 21 are annularly connected to each other to form a circumferential direction unit 23. Bent portions of the zigzag portions 21 of the circumferential direction units 23 are connected to one another through connection portions 25. In this manner, the plural circumferential direction units 23 are connected to one another through the connection portions 25 to thereby form the inner stent 20 shaped generally like a cylinder.

As shown in Fig. 3(b), the inner stent 20 of the cylinder like shape may be formed by processing a metal cylinder so that each of circumferential direction units 23 is formed of plural frame-like portions 22 which are circumferentially connected and so that the circumferential direction units 23 are axially connected to one another through plural connection portions 25.

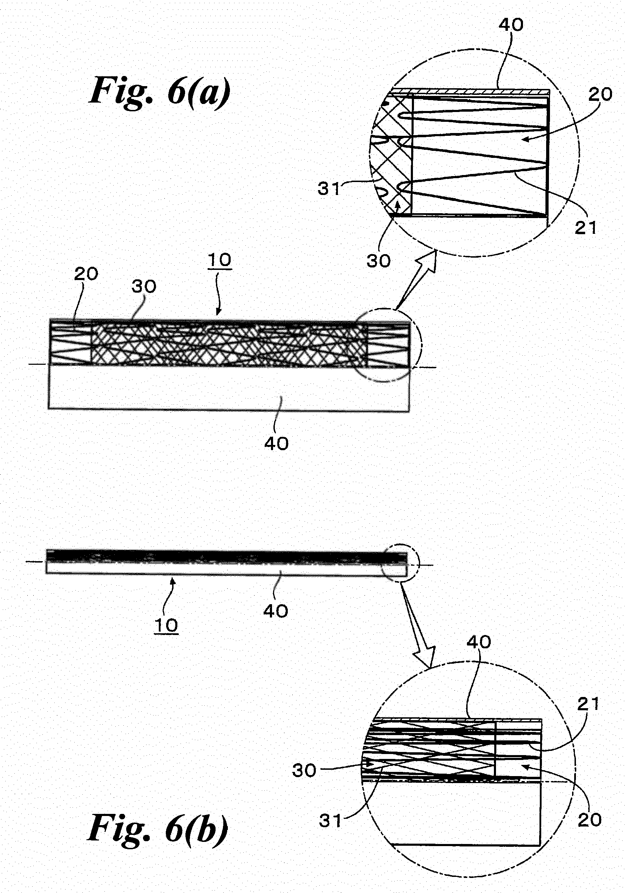

In this embodiment, as shown in Figs. 2 and 6, the axial length of the inner stent 20 is formed to be longer than the axial length of the outer stent 30 so that the inner stent 20 protrudes outward from both axial ends of the outer stent 30 when the outer stent 30 is put on the outer circumference.

The thickness of the metal cylinder forming the inner stent 20 is preferably in a range of 0.05 to 1.0 mm, more preferably in a range of 0.1 to 0.5 mm.

The material of the metal cylinder forming the inner stent 20 is not particularly limited. For example, stainless steel, Ta, Ti, Pt, Au, W or the like or shape memory alloy such as Ni-Ti-based alloy, Co-Cr-based alloy, Co-Cr-Ni-based alloy, Cu-Zn-X (X = Al, Fe or the like) alloy, Ni-Ti-X (X = Fe, Cu, V, Co or the like) alloy, etc. is preferred.

When the aforementioned shape memory alloy is used, the zigzag portions 21 or the frame-like portions 22 are formed in the metal cylinder, and then a shape memory process is applied to the metal cylinder in the diametrically-expanded state. Thus, a self-expansion type inner stent 20 the diameter of which is expanded in the free state can be obtained. Of course, the inner stent 20 may be a balloon dilation type stent the diameter of which is expanded by a balloon catheter.

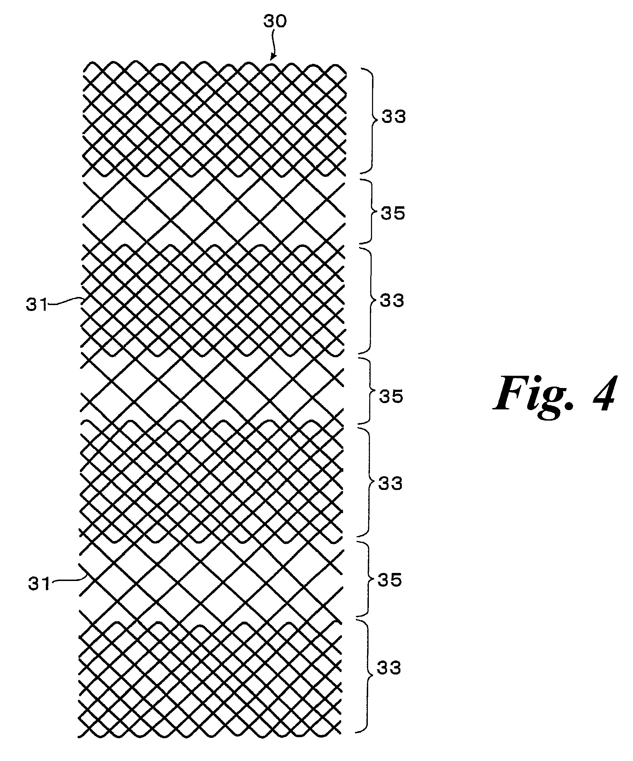

The outer stent 30 put on the outer circumference of the inner stent 20 is formed cylindrically by braiding metal wire 31. As shown in the perspective view of Fig. 1 and the development view of Fig. 4, in the outer stent 30 of this embodiment, portions 33 ("densely braided portions 33") where the metal wire 31 is braided densely and portions 35 ("sparsely braided portions 35") where the metal wire 31 is braided sparsely are formed alternately and the densely braided portions 33 and 33 are disposed in both axial end portions when the outer stent 30 is viewed along the axial direction. However, it is not particularly limited in terms of the method of braiding. Although the number of the sparsely braided portions 35, the number of the densely braided portions 33, etc. are not particularly limited, it is preferable that the densely braided portions 33 are formed at four places and the sparsely braided portions 35 are formed at three places as in this embodiment.

The outer stent 30 is formed by braiding metal wire 31. In the invention, the meaning of "braiding metal wire 31" includes weaving, knitting or entwining metal wire 31, simply crossing metal wires 31, or the like.

Although the metal wire 31 in this embodiment has a circular section, the metal wire 31 may have a polygonal section. The outer diameter of the metal wire 31 is preferably in a range of 0.5 to 80 mm, more preferably in a range of 3 to 30 mm.

The material of the metal wire 31 forming the outer stent 30 is not particularly limited. The same material as that of the aforementioned inner stent 20, for example, stainless steel, Ta, Ti, Pt, Au, W or the like or shape memory alloy such as Ni-Ti-based alloy, Co-Cr-based alloy, Co-Cr-Ni-based alloy, Cu-Zn-X (X = Al, Fe, etc.) alloy or Ni-Ti-X (X = Fe, Cu, V, Co, etc.) alloy is preferred.

Although the outer stent 30 in this embodiment is a self-expansion type stent the diameter of which is expanded in the free state, the outer stent 30 is not particularly limited but may be a balloon dilation type stent the diameter of which is expanded by a balloon catheter.

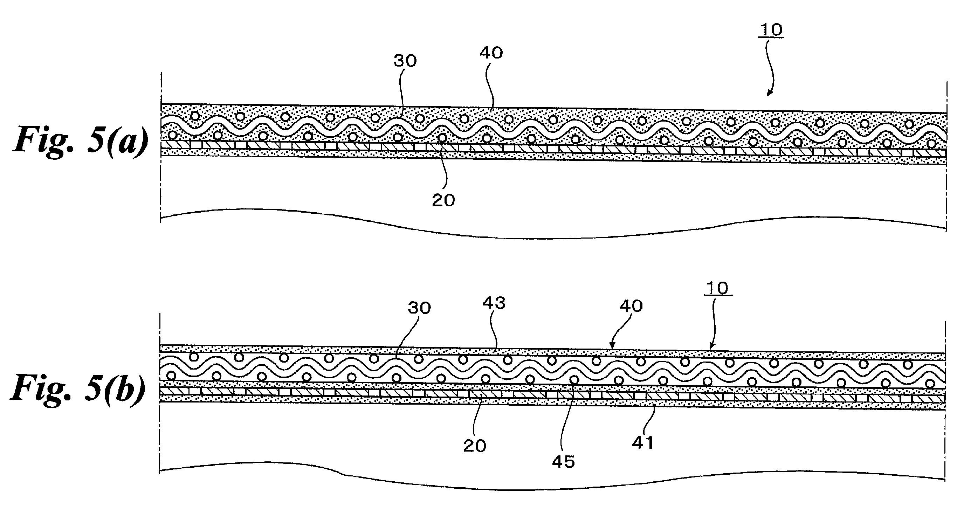

The aforementioned inner stent 20 and outer stent 30 are covered with a cover member 40. As shown in Fig. 5, in this embodiment, the cover member 40 is fixed with a predetermined thickness to wrap the inner stent 20 and the outer stent 30 in the thickness direction so that the inner stent 20 and the outer stent 30 are embedded in the cover member 40 as shown in Fig. 5(a). Thus, the inner stent 20 and the outer stent 30 are fixed to each other by the cover member 40 so as not to be displaced.

As shown in Fig. 6, the cover member 40 is formed on the whole length of the inner stent 20 so that the whole stent 10 is covered with the cover member 40.

For example, polyurethane, silicone, natural rubber, Nylon elastomer, polyether block amide, polyethylene, polyvinyl chloride, vinyl acetate, or a fluorine-based resin such as polytetrafluoroethylene (PTFE), perfluoroalkoxy resin (PFA), ethylene tetrafluoride-propylene hexafluoride copolymer (FEP), ethylene tetrafluoride-ethylene copolymer (ETFE), etc. is preferred as the material of the cover member 40.

It is preferable that the outer circumference of the cover member 40 may be further coated with polybutadiene, styrene-based elastomer or the like so that polyurethane, Nylon elastomer or the like easy to be hydrolyzed can be protected.

In this embodiment, as shown in Fig. 5(a), the inner stent 20 and the outer stent 30 are embedded in the cover member 40. However, as shown in Fig. 5(b), an inner cover 41 may be fixed to the inner circumference of the inner stent 20, an outer cover 43 may be fixed to the outer circumference of the outer stent 30, and a resin fixing layer 45 may be disposed between the inner stent 20 and the outer stent 30 so that the inner stent 20 and the outer stent 30 are fixed to each other through the resin fixing layer 45, in order to improve flexibility of the stent 10.

It is not necessary to use all of the inner cover 41, the outer cover 43 and the resin fixing layer 45. For example, there may be used any of combinations:
(1) only the inner cover 41;
(2) only the outer cover 43;
(3) only the resin fixing layer 45;
(4) both the inner cover 41 and the outer cover 43;
(5) both the inner cover 41 and the resin fixing layer 45; or
(6) both the outer cover 43 and the resin fixing layer 45.

It may be structured such that one axial end side of the inner stent 20 and the outer stent 30 is fixed by the resin fixing layer 45 but the other axial end side thereof is not fixed.

For example, polyurethane, silicone, styrene-based elastomer, polybutadiene, Nylon elastomer, polyether block amide, polyethylene, polyvinyl chloride, vinyl acetate, or a fluorine-based resin such as polytetrafluoroethylene (PTFE), perfluoroalkoxy resin (PFA), ethylene tetrafluoride-propylene hexafluoride copolymer (FEP), ethylene tetrafluoride-ethylene copolymer (ETFE), etc. is preferred as the material of each of the inner cover 41, the outer cover 43 and the resin fixing layer 45.

An example of usage of the stent 10 having the aforementioned structure will be described below.

First, as shown in Fig. 6(b), the stent 10 in the diametrically-contracted state is received in an inner circumference of a distal end portion of a not-shown medical tube such as a catheter, a sheath, etc. Then, a guide wire not shown is inserted into a tubular organ such as a bile duct, a blood vessel, the trachea, etc. percutaneously or incisionally, so that the medical tube containing the stent 10 is conveyed through the guide wire. When the distal end of the medical tube reaches an affected area such as a narrowed area of the tubular organ, the guide wire is pulled out and a pusher or the like is inserted into the medical tube. When the stent 10 is extruded from the distal end of the medical tube through the pusher or the like, the diameter of the stent 10 is expanded as shown in Fig. 6(a). As a result, the narrowed area generated in the tubular organ is forcibly expanded so that the stent 10 is detained.

On this occasion, the stent 10 has the inner stent 20 made of a metal cylinder the total length of which is hardly changed between the diametrically-contracted state and the diametrically-expanded state. Thus, the whole axial length of the stent 10 is substantially unchanged even when the stent 10 changes from the diametrically-contracted state shown in Fig. 6(b) to the diametrically-expanded state shown in Fig. 6(a). Accordingly, shortening can be prevented effectively so that the stent 10 can be detained in a target place of the tubular organ accurately without displacement.

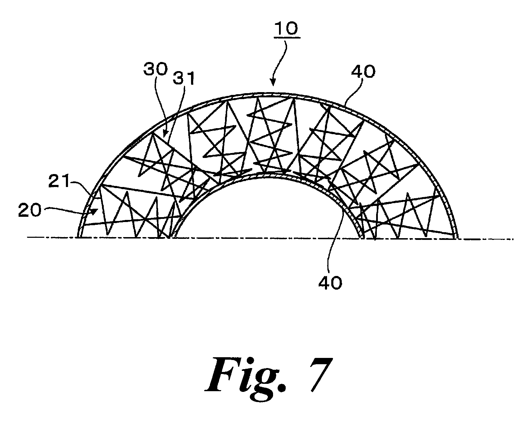

Even when the tubular organ in which the stent 10 should be detained is bent, the stent 10 can surely follow it. That is, even when the stent 10 is detained in a bent tubular organ, while the detained stent 10 is bent along the inner wall of the tubular organ as shown in Fig. 7, since the outer stent 30 formed by braiding metal wire 31 is put on the outside of the inner stent 20 so that corner portions of the zigzag portions 21 or frame-like portions 22 of the inner stent 20 can be prevented from protruding outward. As a result, the tubular organ can be prevented from being damaged while the corner portions can be prevented from being stuck into the inner wall of the tubular organ, and the stent 10 can be exchanged easily.

According to the aforementioned stent 10, there is obtained an excellent effect in which the stent 10 can be prevented from shortening at the time of detention in the tubular organ and the stent 10 can be detained in a target place of the tubular organ without displacement. Further, even when the stent is detained in a bent tubular organ, the metal wire can be prevented from being protruded outward. Such effect can not been obtained in a background-art stent.

As shown in Figs. 1 and 4, it is more preferable that the outer stent 30 has densely braided portions 33 where the metal wire 31 is braided densely, and sparsely braided portions 35 where the metal wire 31 is braided sparsely as in this embodiment when the outer stent 30 is viewed along the axial direction thereof. In this case, because expansion force in the densely braided portions 33 of the outer stent 30 becomes larger than that in the sparsely braided portions 35 so that the densely braided portions 33 protrude more annularly outward than the sparsely braided portions 35, the stent 10 is firmly fixed to the inner wall of the tubular organ so that the stent 10 can be more surely prevented from being displaced.

In the stent 10, the cover member 40 made of a resin layer is fixed to the inner circumference of the inner stent 20 and/or the outer circumference of the outer stent 30.

In this embodiment, as shown in Fig. 5(a), the cover member 40 is fixed so that the circumferential walls of the inner stent 20 and the outer stent 30 are embedded therein in the thickness direction. For this reason, the cover member 40 can prevent a tumor tissue or the like from entering and blocking the inside of the stent 10. Because the cover member 40 is fixed also to the outer circumference of the outer stent 30, the tumor tissue or the like can be prevented from entering a gap between metal wires 31 of the outer stent 30 so that the stent 10 can be recovered.

As shown in Figs. 5(a) and 5(b), when the inner stent 20 and the outer stent 30 are embedded in the cover member 40 or fixed by the resin fixing layer 45, there is obtained an effect in which the two stents 20 and 30 can hardly be displaced so that the stent 10 can be more surely detained in a desired place. In this embodiment, the cover member 40 serves also as the resin fixing layer 45.

The outer stent 30 formed by braiding the metal wire 31 may be largely variable in terms of the length. In the case where one axial end side of the inner stent 20 and the outer stent 30 is fixed by the resin fixing layer 45 but the other axial end side thereof is not fixed, even when the outer stent 30 occasionally becomes very short, the inner stent 20 made of a metal cylinder is prevented from being shortened in accordance with very shortening of the outer stent 30. Accordingly, the effect of preventing shortening of the stent 10 can be fulfilled more surely.

### Reference Signs List

- 10: stent
- 20: inner stent
- 21: zigzag portion
- 22: frame-like portion
- 23: circumferential direction unit
- 25: connection portion
- 30: outer stent
- 31: metal wire
- 33: portion where metal wire is braided densely (densely braided portion)
- 35: portion where metal wire is braided sparsely (sparsely braided portion)
- 40: cover member
- 45: resin fixing layer

## Claims

1. A stent (10), including:
an inner stent (20) which is formed by processing a metal cylinder so that circumferential direction units (23) are axially connected to one another through plural connection portions (25) and so that each of the circumferential direction units (23) is formed of a circumferentially-extended and annularly-connected zigzag portion (21) or of circumferentially-connected plural frame-like portions (22); and
an outer stent (30) which is formed cylindrically by braiding metal wire (31) and which is put on an outer circumference of the inner stent (20),
**characterized in that**
the inner stent (20) and the outer stent (30) are fixed by a resin fixing layer (45).

2. The stent of Claim 1,
wherein the outer stent (30) has
portions where the metal wire is braided densely (33), and
portions where the metal wire is braided sparsely (35), when the outer stent (30) is viewed along the axial direction thereof.

3. The stent of Claim 1 or 2,
wherein a cover member (40) made of a resin layer is fixed to an inner circumference of the inner stent (20) and/or an outer circumference of the outer stent (30).

## Patentansprüche

1. Stent (10), der enthält:
einen inneren Stent (20), der ausgebildet wird, indem ein Metallzylinder so bearbeitet wird, dass Einheiten (23) in Umfangsrichtung über mehrere Verbindungsabschnitte (25) axial miteinander verbunden sind, und so bearbeitet wird, dass jede der Einheiten (23) in Umfangsrichtung aus einem in Umfangsrichtung verlaufenden und ringförmig verbundenen Zickzack-Abschnitt (21) oder aus mehreren in Umfangsrichtung verbundenen rahmenartigen Abschnitten (22) gebildet wird; sowie
einen inneren Stent (30), der zylindrisch ausgebildet wird, indem Metalldraht (31) geflochten wird, und der auf einen Außenumfang des inneren Stent (20) aufgesetzt wird,
**dadurch gekennzeichnet, dass**
der innere Stent (20) und der äußere Stent (30) über eine Harz-Fixierschicht (45) fixiert werden.

2. Stent nach Anspruch 1,
wobei der äußere Stent (30) aufweist:
Abschnitte (33), in denen der Metalldraht dicht geflochten ist, und
Abschnitte (35), in denen der Metalldraht, in der axialen Richtung des äußeren Stent (30) gesehen, locker geflochten ist.

3. Stent nach Anspruch 1 oder 2,
wobei ein Abdeckungselement (40), das aus einer Harzschicht besteht, an einem Innenumfang des inneren Stent (20) und/oder einem Außenumfang des äußeren Stent (30) fixiert ist.

## Revendications

1. Stent (10) comprenant :
un stent interne (20) qui est formé par le traitement d'un cylindre métallique de façon à ce que des unités de direction circonférentielle (23) soient connectées axialement les unes aux autres par l'intermédiaire de plusieurs portions de connexion (25) et de façon à ce que chacune des unités de direction circonférentielle (23) soit constituée d'une portion en zigzag (21), s'étendant sur la circonférence et connectées de manière annulaire, ou de portions en forme de cadres (22) connectées sur la circonférence ; et
un stent externe (30) qui présente une forme cylindrique obtenue par tressage de fil métallique (31) et qui est placé sur une circonférence externe du stent interne (20),
**caractérisé en ce que**
le stent interne (20) et le stent externe (30) sont fixés par une couche de fixation par résine (45).

2. Stent selon la revendication 1,
dans lequel le stent externe (30) comprend
des portions dans lesquelles le fil métallique est tressé de manière serrée (33) et
des portions dans lesquelles le fil métallique est tressé de manière lâche (35) lorsque le stent externe (30) est vu le long de sa direction axiale.

3. Stent selon la revendication 1 ou 2,
dans lequel un élément de couvercle (40) constitué d'une couche de résine est fixé à une circonférence interne du stent interne (20) et/ou une circonférence externe du stent externe (30).
